# EUROPEAN PATENT APPLICATION

(11) **EP 1 361 268 A1**
(43) Date of publication of application: **12.11.2003**
(21) Application number: 02010439.4
(22) Date of filing: 08.05.2002
(51) Int. Cl.: C12N 5/10, C12N 5/08, A61K 35/12, A61P 31/18, A61P 35/00, A61P 37/00

(54) **Pax-5-deficient pro-B cells, methods of producing them and the use of such cells in human therapy**

(71) Applicant: Boehringer Ingelheim International GmbH, 55216 Ingelheim (DE)
(72) Inventor: Busslinger, Meinrad, 1030 Wien (AT); Mikkola, Ingvild, 9100 Kvaloysletta (NO); Heavey, Barry, Athlone (IE)
(74) Representative: Laudien, Dieter, Dr.

(57) **Abstract**

Human pro-B cells deficient in Pax5 expression and methods of producing them. Human Pax5-deficient pro-B cells are useful for the therapy of disorders associated with a depletion of the lymphoid system, in particular for the treatment of immunodeficiencies like AIDS.

SEQUENCE LISTING

<110> Boehringer Ingelheim International GmbH

<120> Pax5-deficient pro-B cells

<130> Case_14_079

<140>
<141>

<160> 30

<170> PatentIn Ver. 2.1

<210> 1
<211> 21
<212> DNA
<213> Artificial Sequence

<220>
<223> Description of Artificial Sequence: PCR Primer

<400> 1
acccaaaagg aaagaaaaac c 21

<210> 2
<211> 21
<212> DNA
<213> Artificial Sequence

<220>
<223> Description of Artificial Sequence: PCR Primer

<400> 2
gtagagcagt gtgagcagga a 21

<210> 3
<211> 21
<212> DNA
<213> Artificial Sequence

<220>
<223> Description of Artificial Sequence: PCR Primer

<400> 3
gtgtttgtgt ggaagactgg a 21

<210> 4
<211> 21
<212> DNA
<213> Artificial Sequence

<220>
<223> Description of Artificial Sequence: PCR Primer

<400> 4
aggaggaaga ggacgatgaa g                                                21

<210> 5
<211> 21
<212> DNA
<213> Artificial Sequence

<220>
<223> Description of Artificial Sequence: PCR Primer

<400> 5
gacaagatga agacccacct g                                                21

<210> 6
<211> 21
<212> DNA
<213> Artificial Sequence

<220>
<223> Description of Artificial Sequence: PCR Primer

<400> 6
ttgctctggg tggcagtaac a                                                21

<210> 7
<211> 23
<212> DNA
<213> Artificial Sequence

<220>
<223> Description of Artificial Sequence: PCR Primer

<400> 7
agagaaaaat tacccgactc ctc                                              23

<210> 8
<211> 23
<212> DNA
<213> Artificial Sequence

<220>
<223> Description of Artificial Sequence: PCR Primer

<400> 8
catccctctt gcgtttgttg gtg                                              23

<210> 9
<211> 20
<212> DNA
<213> Artificial Sequence

<220>
<223> Description of Artificial Sequence: PCR Primer

<400> 9
gggggctata agttctttgc                                                    20

<210> 10
<211> 20
<212> DNA
<213> Artificial Sequence

<220>
<223> Description of Artificial Sequence: PCR Primer

<400> 10
tccaacactt cgagaggtcc                                                    20

<210> 11
<211> 21
<212> DNA
<213> Artificial Sequence

<220>
<223> Description of Artificial Sequence: PCR Primer

<400> 11
gcccgtggag attgagagga c                                                  21

<210> 12
<211> 21
<212> DNA
<213> Artificial Sequence

<220>
<223> Description of Artificial Sequence: PCR Primer

<400> 12
gtgcttggag ttattgtgga c                                                  21

<210> 13
<211> 21
<212> DNA
<213> Artificial Sequence

<220>
<223> Description of Artificial Sequence: PCR Primer

<400> 13
tcccccagag gtcagtgtta c                                                  21

<210> 14
<211> 21
<212> DNA
<213> Artificial Sequence

<220>
<223> Description of Artificial Sequence: PCR Primer

<400> 14
gccagtccaa agtccccaat c                                                21

<210> 15
<211> 29
<212> DNA
<213> Artificial Sequence

<220>
<223> Description of Artificial Sequence: PCR Primer

<400> 15
gcggaattcc cagtcatgaa gaagatgca                                        29

<210> 16
<211> 30
<212> DNA
<213> Artificial Sequence

<220>
<223> Description of Artificial Sequence: PCR Primer

<400> 16
gcgggatccg cagcacttga gtaggttcac                                       30

<210> 17
<211> 28
<212> DNA
<213> Artificial Sequence

<220>
<223> Description of Artificial Sequence: PCR Primer

<400> 17
gcggaattcc accatccctg acggtgaa                                         28

<210> 18
<211> 30
<212> DNA
<213> Artificial Sequence

<220>
<223> Description of Artificial Sequence: PCR Primer

<400> 18
gcgggatccg ggggtgacac taacgaggat                                        30

<210> 19
<211> 30
<212> DNA
<213> Artificial Sequence

<220>
<223> Description of Artificial Sequence: PCR Primer

<400> 19
gcggaattcc tgtggcacgg aacttctagt                                        30


<210> 20
<211> 30
<212> DNA
<213> Artificial Sequence

<220>
<223> Description of Artificial Sequence: PCR Primer

<400> 20
gcgaagcttc ctgtccgaag agtcactatg                                        30


<210> 21
<211> 21
<212> DNA
<213> Artificial Sequence

<220>
<223> Description of Artificial Sequence: PCR Primer

<400> 21
cctgggcatc tgaggacttt t                                                 21


<210> 22
<211> 21
<212> DNA
<213> Artificial Sequence

<220>
<223> Description of Artificial Sequence: PCR Primer

<400> 22
ggggatactg aggctgcttg a                                                 21


<210> 23
<211> 21
<212> DNA
<213> Artificial Sequence

<220>
<223> Description of Artificial Sequence: PCR Primer

<400> 23
agtggcaaag gaggtagaga t                                                 21


<210> 24
<211> 21
<212> DNA
<213> Artificial Sequence

<220>
<223> Description of Artificial Sequence: PCR Primer

<400> 24
aacataggga agcaacagaa t                                                21

<210> 25
<211> 21
<212> DNA
<213> Artificial Sequence

<220>
<223> Description of Artificial Sequence: PCR Primer

<400> 25
caaaagaagt ccagagcagt c                                                21

<210> 26
<211> 21
<212> DNA
<213> Artificial Sequence

<220>
<223> Description of Artificial Sequence: PCR Primer

<400> 26
gggcaaaata gggcatcagt t                                                21

<210> 27
<211> 21
<212> DNA
<213> Artificial Sequence

<220>
<223> Description of Artificial Sequence: PCR Primer

<400> 27
gtgggcaatc agagtcgtca a                                                21

<210> 28
<211> 21
<212> DNA
<213> Artificial Sequence

<220>
<223> Description of Artificial Sequence: PCR Primer

<400> 28
atgtgggagt gctgtggaga t                                                21

<210> 29
<211> 24
<212> DNA
<213> Artificial Sequence

<220>
<223> Description of Artificial Sequence: PCR Primer

<400> 29
catggcgaga agctctttag ttcc                                              24

<210> 30
<211> 24
<212> DNA
<213> Artificial Sequence

<220>
<223> Description of Artificial Sequence: PCR Primer

<400> 30
tgcaaggcga ttaagttggg taac                                              24

## Description

The present invention relates to the field of immunology, in particular to the therapy of disorders associated with defects in the lymphoid system, e.g. immunodeficiencies.

The hematopoietic stem cell gives rise to all lymphoid lineages by differentiating through the intermediary stage of the common lymphoid progenitor (Kondo et al., 1997). Entry of this progenitor into the B cell pathway depends on the transcription factors E2A, EBF and Pax5 (BSAP), which control two separate transcriptional programs (Busslinger et al., 2000). The regulators E2A and EBF coordinately activate the expression of B-cell-specific genes (Sigvardsson et al., 1997; Kee and Murre, 1998), which is essential for the generation of the earliest B cell progenitors (pro-B cells) at the onset of B-lymphopoiesis (Zhuang et al., 1994; Bain et al., 1994; Lin and Grosschedl, 1995). The mere activation of the B-lymphoid expression program is, however, not sufficient for commitment to the B cell lineage, as Pax5-deficient pro-B cells still retain the broad developmental potential of early hematopoietic progenitors (Rolink et al., 1999; Nutt et al, 1999a) despite normal expression of E2A and EBF (Nutt et al., 1997). Instead, Pax5 is essential to repress the promiscuous transcription of lineage-inappropriate genes and to commit progenitor cells to the B cell pathway by suppressing alternative cell fates (Rolink et al., 1999; Nutt et al, 1999a).
Recently, the inventors demonstrated that Pax5 is also essential for maintaining the identity of mature B cells in late B-lymphopoiesis (Horcher et al., 2001). In response to *Pax5* inactivation, mature B cells down-regulated the transcription of many B-cell-specific genes and simultaneously activated the expression of non-B-lymphoid genes (Horcher et al., 2001). However, lineage-tracing experiments failed so far to provide evidence that the mature Pax5-deficient B cells became decommitted and differentiated *in vivo* into other hematopoietic cell types.

Whether the commitment of tissue-specific stem cells to different developmental pathways is a reversible or irreversible process is a fundamental question in the field of organogenesis (Weissman, 2000). This question has recently been investigated for the neural crest stem cell that generates either neurons or glial cells during peripheral nervous system development. In this case, transient activation of Notch was shown to be sufficient to irreversibly commit neural crest stem cells to the glial cell fate (Morrison et al., 2000). The question of reversibility is also of high importance in view of hematologically-based therapies of disorders that are accompanied by immunodeficiencies.

Fundamental to the pathophysiology of the acquired immunodeficiency syndrome (AIDS) is the inability of the immune system to compensate for the depletion of specific immune effector cells caused by HIV-1 (HIV). By targeting the cells responding to it, HIV undermines the immune response favoring viral spread in a self-accelerating manner. Yet, for some individuals the immune system remains vigorous and capable of controlling HIV (Rosenberg et al., 1997).
Anti-retroviral medication can diminish the rapidity of viral spread but does not appear able to restore critical, virus-controlling immunity. Immune recovery observed with anti-retroviral drugs is not sufficient to permit immune control of HIV without medications. Strategies to overcome this problem and to regenerate vigorous HIV-specific immunity focus on two basic goals: 1) To provide additional anti-HIV protection to developing cells; or 2) To enhance the generation of specific T cell subsets. Currently, potent new anti-viral drugs and vaccines are regarded as leading methods to achieve these goals. Autologous cells manipulated ex vivo and adoptively provided to alter the balance in favor of host control of HIV is considered a plausible alternative (Levine et al., 2001).
A number of efforts are ongoing using retroviral transduction of primitive cells. By transduction of genetic constructs to protect cells from HIV infection, enhanced immune reconstitution is hypothesized and currently being tested.
Enhancing T-lymphoid regeneration is an obviously desirable goal that stem cell expansion strategies may ultimately be tailored to achieve. In addition, ex vivo T cell differentiation systems are being developed (Levine et al., 2001).

The ability to achieve T cell neogenesis ex vivo has been difficult except by using organ culture or, with limited success, co-culture systems. There are currently other efforts using three-dimensional matrices that permit single positive CD4⁺ and CD8⁺ cells to emerge from CD34⁺ or AC133⁺ bone marrow cells (Poznansky et al., 2000). While de novo T cell generation has been documented in these systems, the ability to expand this system to a clinical scale is unlikely and so far untested.

Alternative strategies to achieve improved immune function using ex vivo T cell manipulation are also being tested. One such method involves the ex vivo expansion of existing circulating T cells in HIV individuals using a method that results in a HIV-free population of CD4⁺ and CD8⁺ cells (Levine et al., 1996).
A modification of this approach is to transduce the cells during the expansion phase with a chimeric T cell receptor gene (Yang et al., 1997). Clinical studies using this approach have demonstrated successful transduction and survival of the transduced T cells in vivo for up to 6 months (Mitsuyasu et al., 2000).

The above summary shows that immune regeneration is only partially successful with available anti-retroviral strategies, providing protection from most opportunistic infections but failing to achieve the immune control of HIV that is now considered to be possible. Achieving immune control without the need for chronic anti-HIV medications is clearly a goal of enormous value and will require regeneration of the robust HIV-specific immune response seen with acute HIV infection. Further definition of events restricting the immune regeneration of thist response and strategies to overcome these restrictions are an ongoing challenge with tremendous therapeutic potential. Hematologically based therapies using gene-modified cells or ex vivo generated cells have been considered a possible approach (Levine, 2001).

Thus, there is a need for therapies that achieve the regeneration of the lymphoid lineages, in particular the regeneration of T cell-mediated immunity, in acquired immunodeficiencies or in tumor treatments that are accompanied with defects in the lymphoid system.

It was an object of the invention to investigate the molecular mechanisms involved in B-lineage commitment in order to harness these findings for human therapy.

In order to solve the object of the invention, it was sought to find out, by conditional inactivation of *Pax5*, whether B-lineage commitment requires only the transient activation or continuous expression of *Pax5* during early B cell development.

The design of the experiments of the invention was based on the consideration that the inactivation of a commitment factor in an already committed cell could have at least three different phenotypic consequences: The factor-deprived cells may receive confusing signals and undergo cell death. Alternatively, these cells may remain irreversible committed to the selected lineage as evidenced by the transient role of Notch signaling in determining the glial cell fate of neural crest stem cells (Morrison et al., 2000). Finally, the cells may decommit and regain the broad developmental potential of an earlier progenitor cell.

The experiments of the invention have shown that the latter is the case for Pax5-dependent commitment to the B-lymphoid lineage. It was surprisingly found by conditional *Pax5* inactivation in committed pro-B cells that Pax5 is required not only to initiate, but also to maintain its transcriptional program in early B cell development. It was found that, as a consequence of *Pax5* inactivation, previously committed pro-B cells regain the capacity to differentiate into macrophages *in vitro* and to reconstitute T cell development *in vivo* in *RAG2*^{-/-} mice.
Hence, the loss of Pax5 alone is sufficient to reverse B-lineage commitment by converting pro-B cells with a restricted B-lymphoid potential into hematopoietic progenitors with a broad developmental potential.

The findings of the present invention raise the intriguing possibility that wild-type pro-B cells have the ability to differentiate into other hematopoietic cell types by down-regulating *Pax5* expression and thus reversing B cell commitment in response to external signals. In this context it is important to note that the *CD19* gene is a direct Pax5 target (Kozmik et al., 1992), whose expression is entirely dependent on the presence of wild-type Pax5 protein levels (Nutt et al., 1997; Nutt et al., 1998). It was therefore argued that CD19 is not only an indicator of Pax5 activity (Nutt et al., 1999b), but also a decisive marker of B cell commitment (Nutt et al., 1999a; Nutt et al., 1999b). Surprisingly however, CD19⁺ progenitors, isolated from the bone marrow of adult mice, can differentiate upon exposure to appropriate cytokines into dendritic cells (Björk and Kincade, 1998) or macrophages (Montecino-Rodriguez et al., 2001), which was accompanied by down-regulation of CD19 expression (Björk and Kincade, 1998). These observations together with the data suggest that committed B cell progenitors can again broaden their developmental potential by repressing *Pax5* transcription under physiological conditions.

Most importantly, the generation of multipotent hematopoietic progenitors from wild-type pro-B cells is also of medical relevance.

The present invention relates to pro-B cells of human origin. that are deficient in functional Pax5 expression. (In the following, these cells are termed "Pax5-deficient pro-B cells").

The Pax5-deficient pro-B cells of the invention have the capacity of long-term reconstitution, self-renewal and homing to the bone marrow. The Pax5-deficient pro-B cells of the invention have also a broad multilineage potential and the ability to completely restore T cell development.

In a further embodiment, the present invention relates to a method for producing human Pax5-deficient pro-B cells, comprising the steps of
a) isolating mononuclear cells from human tissue,
b) enriching the cell population obtained in a) for lympoid progenitor cells,
c) in vitro differentiating the progenitor cells enriched in b) into pro-B cells and expanding the pro-B cells in culture with appropriate cytokines,
d) identifying and characterizing the pro-B cells according to the expression of B-lymphoid-specific cell surface proteins,
e) inactivating the *Pax5* gene in the pro-B cells,
f) isolating and growing the Pax5-deficient pro-B cells under pro-B cell culture conditions.

In step a), for generating Pax5-deficient pro-B cells of human origin, suitable tissues, from which mononuclear cells can be isolated, are fetal liver, fetal cord blood, bone marrow and adult peripheral blood.

In step b), lymphoid progenitor cells can be enriched by sorting for cells co-expressing the antigens CD34, CD38 and CD10. The CD34⁺CD38⁺CD10⁺ lymphoid progenitor cells are isolated by state-of-the-art cell sorting methods using CyChrome-labeled anti-CD34 (e.g. 8G12), phycoerythrin-labeled anti-CD38 (e.g. T16) and fluorescein isothiocyanate-labeled anti-CD10 (e.g. W8E7) antibodies, which can be obtained from commercial sources (Beckman-Coulter and Becton-Dickinson).

In step c), the sorted CD34⁺CD38⁺CD10⁺ lymphoid progenitors are expanded and differentiated in vitro, usually in co-culture with appropriate human or mouse stromal cells in a pro-B cell medium, an example of which is described in detail in Example 5. Examples for mouse stromal cells are: MS-5 cells (Berardi et al., 1997), ST2 cells (Ogawa et al., 1988) and S17 cells (Cumano et al., 1990).

The human cytokines that are used for pro-B cell culture are usually present in recombinant form: stem cell factor (SCF), interleukin-2 (IL-2), interleukin-15 (IL-15) and thymic stromal lymphopoietin (TSLP). The first three cytokines can be obtained from commercial sources (see Example 5), while recombinant human TSLP (Quentmeier et al., 2001) is specially generated to improve the expansion of human pro-B cells.

In step d), the in vitro cultured pro-B cells are identified by their cell surface phenotype (CD34⁺CD19⁺CD10⁺CD79a⁺CD38⁻) by flow cytometric analysis using appropriately labeled anti-CD79a (e.g. J14.119) and anti-CD19 (e.g. HD37 antibodies) in combination with the anti-CD34, anti-CD10 and anti-CD38 antibodies described above.

In step e), the expression of the *Pax5* gene is inactivated by antisense RNA, small interfering RNA or ribozyme strategies that are described in detail below.

In step f), the Pax5-deficient pro-B cells are identified by the loss of expression of one or more Pax5 target genes, preferably by loss of CD19 expression, which is known to be totally dependent on Pax5 function (Kozmik et al., 1992). The CD34⁺CD19⁻CD10⁺CD79a⁺ pro-B cells lacking Pax5 expression are isolated by cell sorting and expanded in pro-B cell medium as described above under step (c).

As demonstrated in the experiments of the invention, murine Pax5-deficient pro-B cells can be obtained from committed pro-B cells in a two-step procedure. First, the pro B-cells are sorted from the bone marrow of *Pax5*^{*F*/*F*} CreED-30 mice and are then cultured on γ-irradiated stromal cells (ST2) in IL-7 containing IMDM medium. Second, following induction of Cre recombinase activity, the two floxed *Pax5*^{*F*} alleles are inactivated by Cre-mediated deletion of exon 2.

Human pro-B cells can be obtained in a similar manner as murine pro-B cells. The conditions required for the growth and culture of human cells, in particular the growth factors, can be determined empirically. Suitable culture conditions, including the growth factor mix, are described in Example 5. Conditions for culturing human pro-B -cells, that are suitable for the method of the invention, have also been described by Kurosaka et al., 1999; Namikawa et al., 1996; Nishihara et al., 1998; Rawlings et al., 1995; Wolf et al., 1991.

In order for the pro-B cells of the invention to achieve a therapeutic benefit, Pax5 function needs to be stably suppressed. To this end, in step e), the Pax5 function is preferably inactivated by one of the three methods described below. To achieve long-term Pax5 inhibition, techniques are applied that ensure stable expression of inhibiting oligonucleotide molecules. The strategies interfering with Pax5 function employ synthetic oligonucleotides capable of hybridizing with DNA or RNA. A preferred embodiment of the invention involves the application of RNA interference (RNAi). RNAi is the process of sequence-specific post-transcriptional gene silencing initiated by double-stranded RNA that is homologous in sequence to the silenced gene. Small interfering RNA (siRNA) duplexes of 21 to 22 nucleotides were shown to be a new powerful tool for inhibiting gene function in mammalian cells (Elbashir et al., 2001). Sui et al. (2002) and Brummelkamp et al. (2002) have recently reported vector-based systems for stable expression of short interfering RNAs. These systems are based on a vector, in which a synthetic, gene-specific target sequence encoding the siRNA is expressed under the control of a promoter that is suitable for transcription of small, non-coding RNA. The siRNAs are thus produced from the vector following its introduction into mammalian cells by standard transfection protocols, e.g. electroporation, lipofection.

As an alternative to siRNA, antisense oligonucleotides can be used to interfere with the expression of the native Pax5 protein.
Antisense oligonucleotides are short stretches of nucleotides that are complementary to a region of the target mRNA and can specifically suppress expression of that particular transcript. Examples of antisense oligonucleotides and their use in experimental and clinical settings have been recently reviewed (Braasch and Corey, 2002; Agrawal et al., 1998; Galderisi et al., 1999; Gewirtz, 1998). The antisense nucleic acid can take the form of RNA expressed from a vector which has been transfected into the cell or take the form of a DNA or RNA oligonucleotide which can be introduced into cells through a variety of means, e.g. by means of cationic liposomes, cationic porphyrins, fusogenic peptides, and artificial virosomes, or cell permeabilization with streptolysin-O and electroporation. Cationic lipids form stable complexes with oligonucleotides, which exhibit improved cellular uptake (Bennett et al., 1992; Lappalainen et al., 1994), thus resulting in enhanced antisense activity.

Alternatively, Pax5 can be inactivated by means of ribozymes. Similar to antisense oligonucleotides, ribozymes bind to substrate RNA through Watson-Crick base pairing, which leads to sequence-specific cleavage of transcripts. Two types of ribozymes, the hammerhead ribozyme and the hairpin ribozyme, have been extensively studied due to their small size and rapid kinetics. Their application has been recently reviewed in several publications (Hampel, 1998; Vaish et al., 1998; Birikh et al., 1997; Earnshaw and Gait 1997; Kore and Eckstein, 1999).

In the present invention, ribozymes can be imported into the cell by various means, as described above for antisense oligonucleotides, or they can be expressed from a vector, which offers the advantage of continued intracellular production of these molecules (Irie et al. 1999; Smith et al., 1997).

Preferably, the Pax5-inhibiting oligonucleotide molecules are produced from a viral vector. For this purpose, the lentiviral vectors are most preferred, as they have been shown to successfully infect primary cells (Luther-Wyrsch et al., 2001). Furthermore, lentiviral vectors have already proven to be suitable for in vivo gene therapy applications due to the following characteristics (Buchschacher et al., 2000; VandenDriessche et al., 2002):

Since lentiviruses are stably integrated into chromosomal DNA and have little tendency to be epigenetically silenced, they offer the potential for long-term expression. Moreover, lentiviral vectors can efficiently transduce dividing as well as non-dividing cells. The ability to pseudotype lentiviral vectors with the vesicular stomatitis virus G (VSV-G) protein allows for the production of high-titer stocks of stable virus with a broad host range (Emi et al., 1991).
To achieve Pax5 inhibition, expression cassettes encoding Pax5-inhibiting oligonucleotide molecules are inserted into lentiviral vectors (Lever, 1996; Follenzi et al., 2002). These vectors are then transfected by standard methods (e.g. electroporation, lipofection) into specialized packaging cells (Kafri et al., 1999) to generate pseudotyped lentiviruses for infection of human pro-B cells that have been cultured as described in step (c) and Example 5.

Based on the gene function-inhibitory methods described above and the published sequence of the human *Pax5* gene (Adams et al., 1992), the person skilled in the art can determine the Pax5 siRNA, antisense or ribozyme target sequences and construct vectors for transfection or infection of pro-B cells.

In another embodiment, the present invention relates to Pax5-deficient pro-B cells for use in the therapy of disorders associated with a depletion of the lymphoid system, in particular in the treatment of immunodeficiencies, e.g. AIDS, and in the therapy of tumors.

For therapeutic applications, either autologous Pax5-deficient pro-B cells are used or Pax5-deficient pro-B cells that are derived from HLA-matched donors (LeBien, 2000).

During progression of AIDS, the bone marrow becomes pancytopenic possibly due to excessive synthesis of cytokines that interfere with the survival and/or generation of progenitors. Essentially, the bone marrow is massively reduced in cellularity due to constant fever and infections. By repopulating the bone marrow with HLA-matched or autologous Pax5-deficient pro-B cells, the break-down of the lymphoid system (T, NK and DC cells) can be alleviated or partially prevented. Repopulation is achieved after intravenous injection of a single dose of Pax5-deficient pro-B cells in an amount that is sufficient to at least partially restore T cell immunity, which can be monitored by an increase of mature T cells in the peripheral blood. Depending on the severity of the condition and on the body weight of the patient, the dosage usually comprises approximately 10⁷ to 10⁸ cells suspended in isotonic saline solution. Initially, treatment of the patient with antibiotics and immunosuppressive drugs is advisable to avoid possible side effects of the treatment.

The application of Pax5-deficient pro-B cells is also useful in tumor therapies. Chemotherapy of tumor patients often leads to massive side effects in the hematopoietic system. The cell numbers of various different lineages are massively reduced. In cancer therapy, Pax5-deficient pro-B cells can be used for transient counteraction of iatrogenic effects during tumor therapy. Injection of Pax5-deficient pro-B cells is beneficial for the rapid recovery of the lymphoid system until the endogenous HSC itself is recovering from the insult.

Alternatively, Pax5-deficient pro-B cells can be used in non-myeloablative stem cell therapy.

Non-myeloablative therapy has recently been developed as a less toxic alternative to myeloablation, the use of which is limited because of severe acute adverse effects. Non-myeloablative therapy was developed on the theory that abolition of abnormal cells may not solely depend on prior myeloablation by high-dose irradiation therapy. It has been suggested that engraftment of donor cells may also help to destroy malignant cells in the recipient. It is argued that conditioning regimens need not be myeloablative, provided that patients receive enough immunosuppressive treatment to allow donor engraftment. Non-myeloablative therapy was, inter allia, successfully applied for metastatic kidney cell carcinoma (Childs et al., 1999; Childs et al., 2000). In this therapeutic approach, bone marrow cells (enriched for stem cells) of an HLA-matched donor are injected into tumor patient who is irradiated at a low dose (approximately one sixth of lethal dose) prior to cell injection. Four weeks later, most bone marrow cells of the patient are of donor origin. As HLA matching is never perfect, some minor loci have not been perfectly matched. These minor loci are considered to be responsible for a graft-versus-tumor reaction that may lead to the rejection of the tumor.

As an alternative to the currently used protocols that use HLA-matched bone marrow cells enriched for stem cells, the Pax5-deficient pro-B cells of the invention may be applied.

Non-myeloablative therapy may be associated with severe adverse effects, because it relies on immunosuppressive treatment to prevent graft-versus-host disease after transplantation. Such treatment predisposes recipients to infection and may also reduce the anti-malignancy effects of donor cells. Although these negative effects are considered to be acceptable because they are outweighed by the benefits of preserving host marrow function until donor marrow engrafts, the application of Pax5-deficient cells in non-myeloablative therapy provides the additional advantage to alleviate these effects.

In a further embodiment, the invention relates to Pax5-deficient pro-B cells that are modified in such a way that, in addition to Pax5, other functions are either partially suppressed or completely inactivated. The modification of the cells may also be such that one or more gene functions are activated.

In a preferred embodiment, the Pax5-deficient pro-B cells are modified in order to prevent HIV entry or intracellular HIV propagation.

HIV-1 infection of susceptible cells is mediated by the specific interaction of viral envelope glycoproteins with the cell surface CD4 receptor and a chemokine coreceptor, CCR5 or CXCR4. Since CCR5 has been shown to be a non-essential co-receptor, it is considered an ideal function to be targeted for preventing HIV infection.

Hence, in a preferred embodiment, the Pax5-deficient pro-B cells are additionally made defective in CCR5 expression.

Based on the published CCR5 sequence (see e.g. Raport et al., 1996), CCR5 expression can be inhibited in a similar manner as described above for the suppression of Pax5 function, e.g. by siRNAs, antisense oligonucleotides or ribozymes. Pax5 function and CCR5 function may be suppressed by introducing two different inhibiting constructs into pro-B cells, e.g. RNA inhibiting molecules targeted to Pax5 on the one hand and to CCR5 on the other hand, or they may be co-repressed by designing a construct encoding both Pax5- and CCR5-inhibiting RNA molecules and importing this molecule into pro-B cells according to one of the methods described above for the inhibition of Pax5 function.

By way of example, a construct containing an anti-CCR5 ribozyme heterotrimer targeted to three different cleavage sites in CCR5 mRNA can be introduced into the Pax5-deficient pro-B cells by retroviral vectors as described by Bai et al. (2001). These authors have shown that cells stably transduced with an anti-CCR5 heterotrimer showed a marked reduction in the surface expression of CCR5 and a concomitant 70% reduction in macrophage-tropic viral infection.

In addition to sequences inhibiting CCR5 co-receptor function, the RNA-inhibiting constructs, e.g. ribozymes, may carry anti-viral sequences, e.g. anti-HIV-1 tat-rev sequences, as described by Bai et al., 2001. Ribozyme heterodimers carrying such sequences in addition to anti-CCR5 sequences showed a similar inhibition of CCR5 surface expression and reduced infectability. It was shown that trimeric and multimeric ribozyme constructs, when transduced into CD34⁺ hematopoietic progenitor cells, conferred resistance to HIV-1 infection (Bai et al., 2001).

In a further embodiment, the invention relates to a pharmaceutical composition containing Pax5-deficient pro-B cells of human origin. The Pax5-deficient cells can be formulated according to methods known in the art, e.g. as known for stem stell therapy. By way of example, autologous Pax5-deficient pro-B cells or Pax5-deficient pro-B cells derived from a donor that is HLA-matched to the patient, are suspended in isotonic saline solution.

### Brief description of the figures:

Fig. 1: Gene expression changes in pro-B cells following *Pax5* inactivation
Fig. 2: *In vitro* differentiation of *Pax5*^{/} pro-B cells into macrophages
Fig. 3*: Pax5*^{/} pro-B cells reconstitute T cell development in *RAG2*^{-/-} mice
Fig. 4*: Pax5*^{+/-} pro-B cells decommit upon switching of expression from the wild-type to the targeted *Pax5* allele

If not otherwise stated, the following materials and methods were used in the Examples:

### Transgenic mice.

*Pax5*^{+/-}, *Pax5*^{*F*/*F*}, *RAG2*^{-/-} and *CreED-30* mice were maintained on the C57BL/6x129/Sv background and genotyped as described (Horcher et al., 2001; Urbanek et al., 1994; Schwenk et al., 1998; Shinkai et al., 1992).

### Pro-B cell culture.

B220⁺ pro-B cells were sorted from the bone marrow of *Pax5*^{+/-} or *Pax5*^{*F*/*F*} CreED-30 mice and cultured on -irradiated ST2 cells in IL-7 containing IMDM medium as described (Nutt et al., 1997). *Pax5*^{+/-} pro-B cell clones were generated by sorting single cells from a CD19⁻ *Pax5*^{+/-} pro-B cell pool with a FACSVantage TSO flow-cytometer (Becton-Dickinson).

### Antibodies and flow cytometry.

Single-cell suspensions were stained and analysed on a FACSCalibur flow-cytometer (Becton-Dickinson) as described (Urbanek et al., 1994). The following fluorescein isothiocyanate (FITC)-, phycoerythrin (PE)- or allophycocyanin (APC)-labelled antibodies were purchased from PharMingen (San Diego, CA): anti-B220 (RA3-6B2), anti-CD4 (L3T4), anti-CD8 (53-6.7), anti-CD19 (1D3), anti-Mac-1 (M1/70), anti-IgM (M41.42), anti-TCRβ (H57-597) and anti-human CD2 (RPA-2.10) antibodies. The PE-anti-F4/80 (C1:A3-1) antibody were obtained from Serotec (Oxford, England). β-Galactosidase activity was measured by loading the pro-B cells with the fluorogenic substrate 5-chloromethylfluorescein di-β-D-galactopyranoside (CMFDG, Molecular Probes) as described (Nutt et al., 1999c).

### Kinetic analysis of Cre-mediated Pax5 inactivation.

B220⁺ pro-B cells were sorted from the bone marrow of 4-week-old *Pax5*^{*F*/*F*} CreED-30 mice, expanded *in vitro* and then treated with 1 µM 4-hydroxy-tamoxifen (OHT; Research Biochemicals International, Natick, MA) for 3 days followed by subsequent culturing in the absence of OHT. The CD19⁻ *Pax5*^{/} pro-B cells were purified to homogeneity by eliminating the few non-deleting CD19⁺ *Pax5* ^{*F*/*F*} pro-B cells by FACS sorting. The kinetic experiments were performed with freshly isolated *Pax5*^{*F*/*F*} *CreED-30* pro-B cells that were expanded *ex vivo* to 30x10⁶ cells for only 9 days prior to OHT treatment as described above. Pro-B cells (6.5x10⁶) were withdrawn at daily intervals after OHT addition. DNA isolated from 0.5x10⁶ cells was analysed by PCR genotyping (Horcher et al., 2001) to confirm deletion of *Pax5* exon 2. Total RNA was prepared from 5x10⁶ cells, using the Trizol Reagent (Gibco-BRL, Life Technologies). Reverse transcription (with random hexamer primers) and semiquantitative PCR were performed as described (Horcher et al., 2001; Nutt et al., 1999c).

The following primers (5' - 3') and PCR conditions were used:

| **gene** | **SEQ ID NO:** | **annealing °C** | **cycles** | **size(bp)** |
|---|---|---|---|---|
| Igδₘ | 1 and 2 | 55 | 25 | 595 |
| Igµₘ | 3 and 4 | 55 | 25 | 513 |
| J-chain | 5 and 6 | 55 | 30 | 338 |
| Pax5 | 7 and 8 | 55 | 25 | 593(FL) 427(E2) |
| HPRT | 9 and 10 | 55 | 25 | 313 |
| EBF | 11 and 12 | 55 | 30 | 481 |
| M-CSF-R | 13 and 14 | 57 | 30 | 1200 |
| CD19 | 15 and 16 | 59 | 25 | 847 |
| mb-1 | 17 and 18 | 59 | 30 | 820 |
| B291 | 19 and 20 | 59 | 30 | 243 |
| CIITA | 21 and 22 | 59 | 30 | 764 |
| rEST1 | 23 and 24 | 57 | 25 | 492 |
| rEST2 25 and 26 | | 57 | 25 | 680 |
| rEST3 27 and 28 | | 57 | 30 | 595 |
| Pax5-lacZ 29 and 30 | | | | |

The PCR products were separated on agarose gels and visualized by ethidium bromide staining. Protein samples were prepared by directly dissolving and boiling 1x10⁶ cells in 80 µl of 2x SDS sample buffer. Total protein (10 µl) was separated by 10% SDS-PAGE and analyzed by Western blotting with a rabbit polyclonal antibody directed against the Pax5 paired domain (Adams et al., 1992).

### In vitro macrophage differentiation.

*Pax5*^{/} pro-B cells were cultured on ST2 cells in the absence of IL-7 for ∼10 days followed by terminal differentiation in IMDM medium containing M-CSF (25 ng/ml; R&D Systems). The phagocytic activity of differentiated cells was determined by incubation with FITC-labelled heat-inactivated *E. coli* (Molecular Probes) as described (Nutt et al., 1999a)).

### Pro-B cell transfer into RAG2^{-/-} mice.

*RAG2*^{-/-} mice at 8-12 weeks of age were γ-irradiated with 550 rad and intravenously injected with 1-5x10⁶ *in vitro* cultured *Pax5*^{/}, *Pax5*^{*F*/*F*} or *Pax5*^{+/-} pro-B cells as described (Rolink et al., 1999). Prior to injection, the *Pax5*^{+/-} pro-B cells were infected with the pMI retrovirus expressing a human CD2 protein lacking its intracellular domain (Deftos et al., 1998).

### Example 1

B-lineage commitment requires continuous expression of *Pax5* during early B cell development

To address the question whether B-lineage commitment requires only the transient activation or continuous expression of *Pax5* during early B cell development, the inventors took advantage of the *Cre-loxP* system for conditional inactivation of a floxed (F) *Pax5*^{*F*} allele carrying *loxP* sequences on either side of the paired domain exon 2 (Horcher et al., 2001). B cell development was normal in *Pax5*^{*F*/*F*} mice, whereas *Pax5*^{/} mice, generated by germline deletion () of exon 2 (Horcher et al., 2001), were phenotypically indistinguishable from *Pax5*^{-/-} mice (Urbanek et al., 1994).

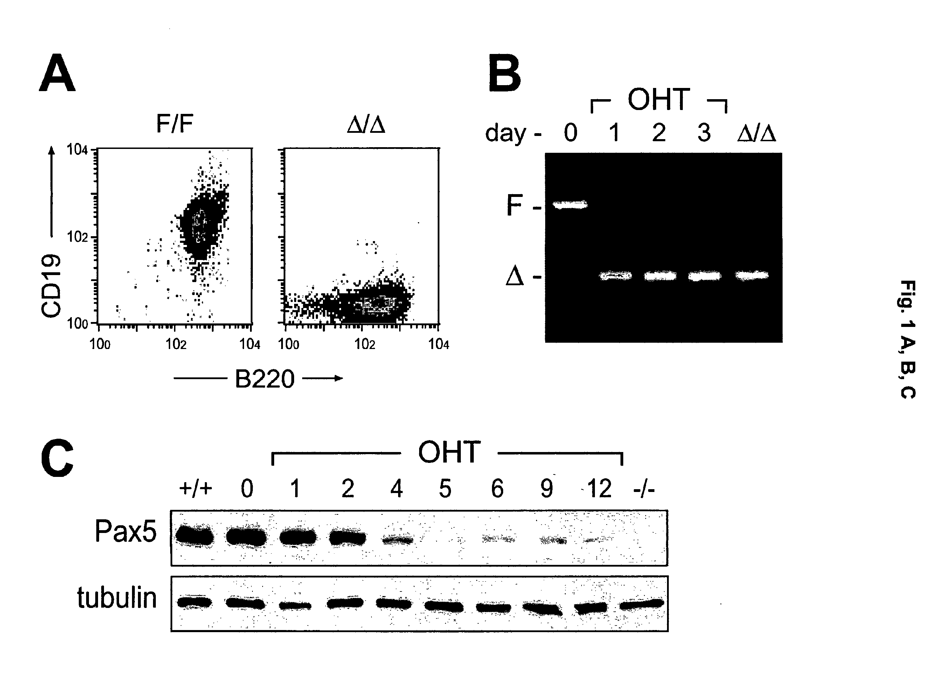

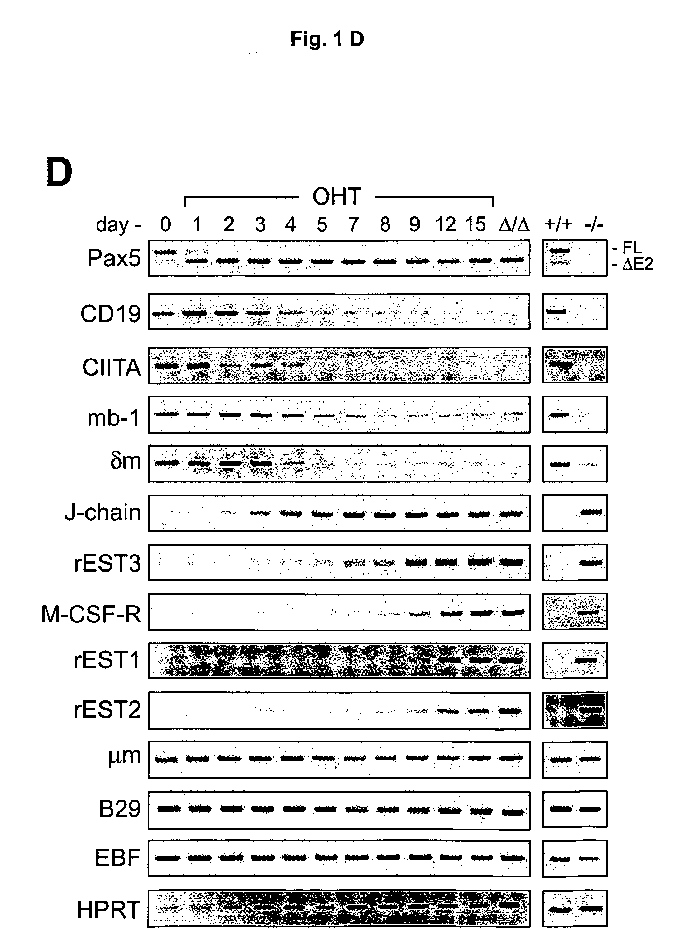

For conditional *Pax5* inactivation at the pro-B cell stage, *Pax5*^{*F*/*F*} mice were crossed with the transgenic line *CreED-30*, which expresses the Cre-EBD[G521R] fusion protein under the control of the immunoglobulin Eµ enhancer and SV40 promoter in the B-lymphoid lineage (Schwenk et al., 1998). The Cre-EBD[G521R] protein consists of the Cre recombinase linked to a mutant human oestrogen receptor ligand-binding domain (EBD) that is insensitive to the natural ligand β-oestradiol, but is responsive to the synthetic antagonist 4-hydroxy-tamoxifen (OHT). This posttranslational induction system allows the specific induction of Cre recombinase activity in B-lymphocytes by OHT treatment (Schwenk et al., 1998). Therefore pro-B cells were sorted from the bone marrow of *Pax5*^{*F*/*F*} CreED-30 mice and cultured these cells *in vitro* on stromal cells in the presence of IL-7. Flow cytometric analysis revealed that these pro-B cells express the B cell markers B220 and CD19 (Fig. 1A, F/F). As transcription of the target gene *CD19* is strictly dependent on Pax5 function (Nutt et al., 1997; Nutt et al., 1998), these data demonstrate that the CD19⁺ *Pax5*^{*F*/*F*} pro-B cells express normal levels of Pax5 and have thus undergone commitment to the B-lymphoid lineage (for further arguments, see below). Treatment of these pro-B cells with 1 µM OHT resulted in the specific loss of CD19 expression, indicating that the *CreED-30* transgene can be used for efficient inactivation of the floxed *Pax5* allele under the *in vitro* culture conditions used (Fig. 1A, /). It is important to note that the proliferation of the pro-B cells remained largely unaffected by the OHT treatment (data not shown). Hence, the OHT-induced inactivation of *Pax5* did result neither in a significant cell loss nor in the outgrowth of a minor subpopulation of *Pax5*^{/} pro-B cells.
Time course analyses revealed that *Pax5*^{*F*/*F*} *CreED*-30 pro-B cells efficiently deleted the *Pax5* exon 2 within 1 day of OHT treatment (Fig. 1B). Within the same short period, the pro-B cells predominantly expressed the truncated *Pax5* transcript lacking exon 2 (Fig. 1D) in agreement with the fact that the *Pax5* mRNA has a short half-life of ∼2 hours (Nutt et al., 1999c). In contrast, the more stable Pax5 protein was reduced to a low level only 4 days after OHT addition (Fig. 1C). Next used RT-PCR analysis was used to investigate the gene expression changes caused by *Pax5* inactivation. One class consisted of the control genes, encoding the ubiquitous *HPRT* mRNA and the B-cell-specific *EBF, B29* (*Ig*β) and µ_{*m*} transcripts, which were neither differentially expressed in wild-type and *Pax5*^{-/-} pro-B cells nor affected by conditional Pax5 loss (Fig. 1D). In contrast, the expression of target genes, which are normally activated by Pax5 in pro-B cells, started to decline at day 4, concomitant with the loss of Pax5 protein (Fig. 1D). These target genes code for the cell surface protein CD19 and Igα (mb-1) (Nutt et al., 1998), the transcription factor CIITA (Horcher et al., 2001) and the alternatively spliced *δ*_{*m*} transcript of the immunoglobulin *IgH* gene (Horcher et al., 2001). Conversely, the genes that are normally silenced by Pax5 at the onset of B cell development were derepressed upon *Pax5* inactivation (Fig. 1D). This class of genes codes for the secreted J-chain (Rinkenberger et al., 1996), the M-CSF receptor (Nutt et al., 1999a) and transcripts of unknown function. Hence, the expression pattern of *Pax5*^{*F*/*F*} pro-B cells appears to be indistinguishable from that of *Pax5*^{+/+} pro-B cells, but can be converted by *Pax5* inactivation into that of *Pax5*^{-/-} pro-B cells. It was therefore concluded that the Pax5-dependent gene expression program is reversible in pro-B cells undergoing *Pax5* inactivation.

Figure 1 shows gene expression changes in pro-B cells following *Pax5* inactivation. (**A**) Loss of CD19 expression upon Cre-mediated deletion of *Pax5*. *Pax5*^{*F*/*F*} CreED-30 pro-B cells were analysed by flow cytometry with PE-anti-CD19 and APC-anti-B220 antibodies either before (F/F) or 5 weeks after treatment (/) with 4-hydroxy-tamoxifen (OHT; 1 µM). (**B**) Deletion kinetics. The loss of the floxed exon 2 of *Pax5* was monitored by PCR analysis of DNA prepared from OHT-treated *Pax5*^{*F*/*F*} CreED-30 pro-B cells. (**C**) Loss of Pax5 protein. Whole cell lysates of OHT-treated *Pax5*^{*F*/*F*} *CreED-30* pro-B cells were analysed by sequential Western blotting with polyclonal anti-Pax5 and monoclonal anti-tubulin antibodies. (D) Semiquantitative RT-PCR analysis. Transcripts of the indicated genes were analysed by RT-PCR at the indicated days after OHT-mediated induction of Cre activity. PCR products were visualised on agarose gels by ethidium bromide staining. "FL" denotes the full-length transcript of the *Pax5*^{*F*} allele and " E2" denotes the *Pax5* transcript lacking exon 2. rEST1, rEST2 and rEST3 refer to Pax5-repressed (r) transcripts of unknown function that were identified by cDNA microarray analysis. The sequences of the µ_{*m*} and *δ*_{*m*} transcripts were amplified from the third or first constant exon to the first transmembrane exon, respectively. Hence, the 5' end of these transcripts has not been determined and could correspond to the Iµ, µ° or Dµ transcript.

### Example 2

### Pax5-deficient pro-B cells adopt again the broad developmental potential of early hematopoietic progenitor cells

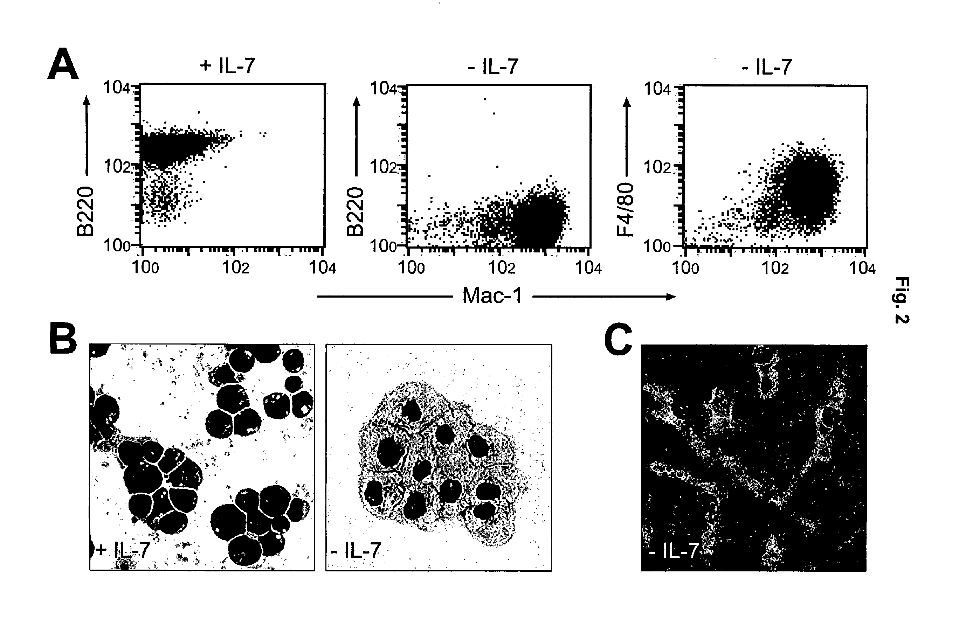

As the *Pax5*^{/} pro-B cells were directly derived from committed *Pax5*^{*F*/*F*} pro-B cells, it was next investigated whether the *Pax5*^{/} pro-B cells remained committed to the B cell pathway or adopted again the broad developmental potential of early hematopoietic progenitor cells. The potential of the *Pax5*^{/} pro-B cells to differentiate into macrophages was assessed by culturing them in the absence of IL-7 on stromal ST2 cells, which abundantly produce the myeloid cytokine M-CSF (Nutt et al., 1999a). *Pax5*^{/} cells, that were differentiated under these conditions for up to 3 weeks, down-regulated the B cell surface protein B220, expressed the macrophage markers Mac-1 and F4/80 (Fig. 2A) and displayed the characteristic morphology of enlarged vacuolar macrophages (Fig. 2B). These differentiated cells furthermore phagocytosed fluorescently labeled *E. coli* (Fig. 2C), indicating that the *Pax5*^{/} pro-B cells can differentiate into mature macrophages like the *Pax5*^{-/-} pro-B cells (Nutt et al., 1999a).

Figure 2 demonstrates the *in vitro* differentiation of *Pax5*^{/} pro-B cells into macrophages.
(A) Flow cytometric analysis. *Pax5*^{/} pro-B cells were cultured for 18 days on ST2 cells in the presence or absence of IL-7. (**B**) May-Grünwald-Giemsa staining of cytospin preparations of *Pax5*^{/} pro-B cells that were grown for 3 weeks on ST2 cells with or without IL-7. (**C**) Phagocytosis. *In vitro* differentiated *Pax5*^{/} macrophages were incubated with FITC-labelled *E. coli*, while the nuclei were stained with DAPI.

### Example 3

### Pax5 deficient pro-B cells have the potential to develop into T-lymphocytes

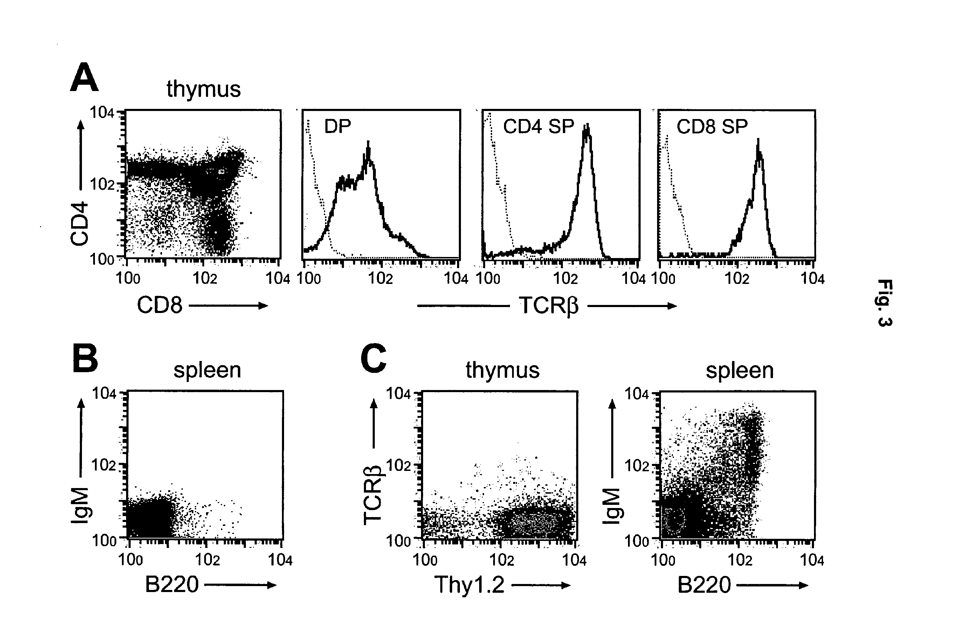

The potential of *Pax5*^{/} pro-B cells to develop into T-lymphocytes was analyzed by intravenous injection into sublethally irradiated *RAG2*^{-/-} mice, which have an early block in T and B cell development due to the absence of V(D)J recombination (Shinkai et al., 1992). Three weeks after cell transfer, the cellularity of the thymus was considerably increased due to the presence of CD4⁺CD8⁺ (double-positive) as well as CD4⁺CD8⁻ and CD8⁺CD4⁻ (single-positive) thymocytes (Fig. 3A). These thymocyte subpopulations were derived from the injected *Pax5*^{/} pro-B cells, as they expressed the T cell receptor (TCR) β similar to wild-type thymocytes (Fig. 3A). Hence, the *Pax5*^{/} pro-B cells could fully reconstitute T cell development, but failed to generate IgM⁺ B cells in the spleen of *RAG2*^{-/-} mice (Fig. 3B) in analogy to *Pax5*^{-/-} pro-B cells (Rolink et al., 1999). The opposite situation was observed for the *Pax5*^{*F*/*F*} pro-B cells, which failed to give rise to TCRβ⁺ thymocytes, but developed into IgM⁺ B cells in the spleen of injected *RAG2*^{-/-} mice (Fig. 3C). Hence, the *Pax5*^{*F*/*F*} pro-B cells are fully committed to the B cell pathway. It was therefore concluded that the inactivation of *Pax5* alone is sufficient to revert the narrow B-lymphoid potential of *Pax5*^{*F*/*F*} pro-B cells to the broad multilineage potential characteristic of early hematopoietic progenitor cells. Figure 3 shows how *Pax5* pro-B cells reconstitute T cell development in *RAG2*^{-/-} mice. (A) Thymus reconstitution by *Pax5*^{/} pro-B cells. 10⁶ *Pax5*^{/} pro-B cells were injected into 550 rad-irradiated *RAG2*^{-/-} mice. Three weeks after transfer, the thymus consisted of 80x10⁶ cells (compared to 5x10⁶ cells of uninjected control *RAG2*^{-/-} mice). The CD4⁺CD8⁺ double-positive (DP), CD4⁺ and CD8⁺ single-positive (SP) thymocytes were analyzed by three-color flow cytometry using FITC-anti-CD4, PE-anti-CD8 and APC-anti-TCRβ antibodies. The three subpopulations (gated on CD4/CD8 expression) exhibited the expected levels of TCRβ expression. Stippled lines indicate the absence of TCRβ staining in control *RAG2*^{-/-} thymocytes. (**B**) Failure of *Pax5*^{/} pro-B cells to differentiate into B cells. The spleen of the same injected *RAG2*^{-/-} mouse shown in (A) was analysed by flow cytometry using APC-anti-B220 and PE-anti-IgM antibodies. (C) Commitment of *Pax5*^{*F*/*F*} pro-B cells to the B-lymphoid lineage. B220⁺ pro-B cells were sorted from the bone marrow of *Pax5*^{*F*/*F*} mice and cultured *in vitro* for only 2 weeks prior to injection of 5x10⁵ cells into 550 rad-irradiated *RAG2*^{-/-} mice. After 3 weeks, the thymus and spleen were analyzed by flow cytometry. The Thy1.2⁺TCRβ⁻ cells in the thymus correspond to the pro-T cells of the *RAG2*^{-/-} host.

### Example 4

### Decommitment of Pax5^{+/-} pro-B cells upon switching of expression from the wild-type to the targeted Pax5 allele

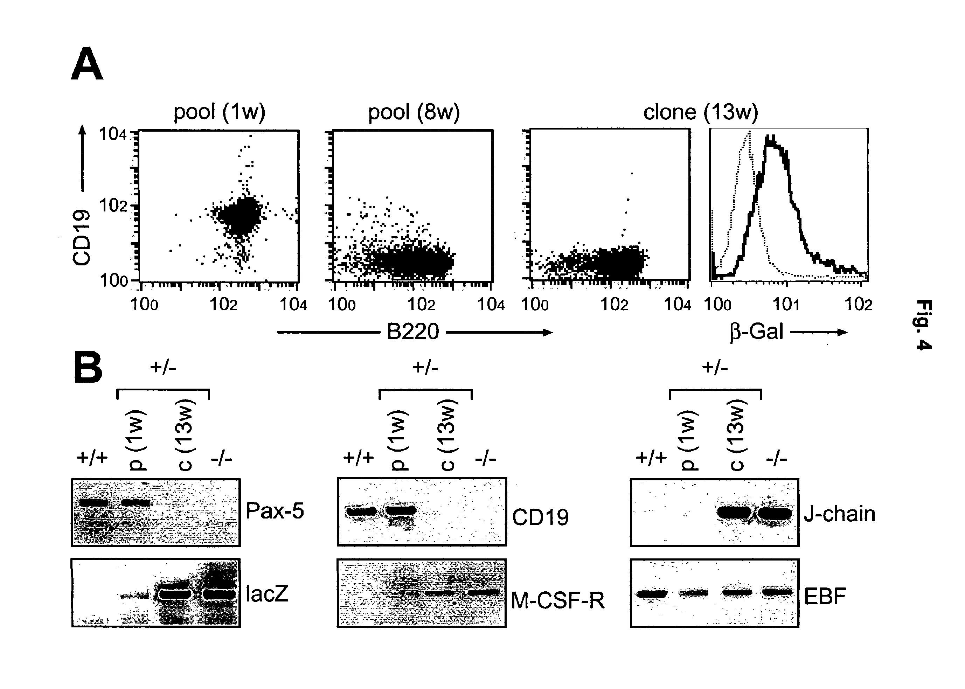

The heterozygous *Pax5*^{+/-} mouse provided a second opportunity to investigate the reversibility of Pax5-dependent B-lineage commitment, as the pro-B cells of this mouse preferentially express only one of the two *Pax5* alleles (Nutt et al., 1999c). Interestingly, heterozygous CD19⁺ pro-B cells are able to switch expression from the wild-type *Pax5* allele to the targeted *Pax5*^{*lacZ*} *(-)* allele during *in vitro* culture, which may reflect a proliferative advantage of the *Pax5* null phenotype for the *in vitro* propagation of pro-B cells (Nutt et al., 1999b; Nutt et al., 1999c). This allele switching results in the loss of expression of the Pax5 target gene *CD19* and simultaneous activation of the *lacZ* gene of the targeted *Pax5* locus (Nutt et al., 1999b; Nutt et al., 1999c) (Fig. 4A). Therefore, CD19⁻ β-Gal⁺ pro-B cell clones were derived from *Pax5*^{*+*}^{/-} bone marrow (Fig. 4A) and demonstrated by RT-PCR analysis that these pro-B cells switched off the expression of activated Pax5 target genes and derepressed genes normally suppressed by Pax5 (Fig. 4B; data not shown). Hence, the CD19⁻ β-Gal⁺ pro-B cells apparently reversed their gene expression program to that of homozygous *Pax5*^{-/-} cells, although, as genotypically heterozygous cells, they still have the ability to reactivate the wild-type *Pax5* allele. These CD19⁻ *Pax5*^{+/-} pro-B cells were also decommitted, as they differentiated, upon IL-7 withdrawal into functional Mac-1⁺F4/80⁺ macrophages with high phagocytic activity, similar to *Pax5*^{-/-} cells (Fig. 4C,D; data not shown). To study the T-lymphoid potential, the CD19⁻ *Pax5*^{+/-} pro-B cells were first infected with a human (h) CD2-expressing retrovirus prior to injection into sublethally irradiated *RAG2*^{-/-} mice. Three weeks after cell transfer, the thymus contained CD4⁺CD8⁺, CD4⁺CD8⁻ and CD8⁺CD4⁻ T cells of donor origin (hCD2⁺), indicating that the CD19⁻ *Pax5*^{+/-} pro-B cells are also able to reconstitute T cell development (Fig. 4E). Thymic reconstitution was still observed 11 weeks after cell transfer due to long-term engraftment of the CD19⁻B220⁺ *Pax5*^{*+*}^{/-} pro-B cells in the bone marrow, from where these progenitors continuously seed the thymus (Fig. 4F, data not shown). In addition to these CD19⁻B220⁺ pro-B cells, the bone marrow contained CD19⁺B220⁺ B-lymphocytes of donor origin (hCD2⁺), which have re-entered the B cell pathway due to activation of the wild-type *Pax5* allele (Fig. 4F). Finally, the bone marrow cells also contained a third hCD2⁺CD19⁻B220⁻ cell population (Fig. 4F), which primarily consisted of mature CD4⁺ and CD8⁺ T-lymphocytes (data not shown). In summary, heterozygous *Pax5*^{+/-} pro-B cells did not only regain the broad developmental potential of early hematopoietic progenitors upon switching to the mutant *Pax5* allele, but at the same time maintained their competence to undergo B cell development by reactivating the wild-type *Pax5* allele *in vivo*.
Fig. 4 shows that *Pax5*^{+/-} pro-B cells decommit upon switching of expression from the wild-type to the targeted *Pax5* allele.
(**A**) Generation of CD19⁻ *Pax5*^{*+*}^{/-} pro-B cell clones. CD19⁺ pro-B cells were isolated from the bone marrow of *Pax5*^{+/-} mice and cultured in the presence of ST2 cells and IL-7 for 8 weeks (w) prior to the generation of single-cell clones by FACS sorting. The pool and a derived clone of *Pax5*^{+/-} pro-B cells was analyzed by flow cytometry with PE-anti-CD19 and APC-anti-B220 antibodies at the indicated times after the start of *in vitro* culture. The cloned *Pax5*^{+/-} pro-B cells were separately analyzed for β-galactosidase activity after loading with the fluorogenic substrate CMFDG. The stippled line indicates wild-type pro-B cells that were similarly treated with CMFDG. (**B**) Semiquantitative RT-PCR analysis. Expression of the indicated genes was determined by RT-PCR in wild-type (+/+), homozygous (-/-) and heterozygous (+/-) *Pax5* mutant pro-B cells. The pool (p) and a clone (c) of *Pax5*^{+/-} pro-B cells were analyzed after 1 and 13 weeks (w) of *in vitro* culture, respectively. (**C,D**) *In vitro* differentiation of CD19⁻ *Pax5*^{+/-} pro-B cells into macrophages. Cloned CD19⁻ *Pax5*^{+/-} pro-B cells were cultured for 3 weeks on ST2 cells in the presence or absence of IL-7 and then analyzed by flow cytometry with PE-anti-B220 and APC-anti-Mac-1 antibodies (C). The phagocytic activity of the differentiated cells was measured by incubation with FITC-labeled *E. coli* (D). (**E**) Thymus reconstitution by CD19⁻ *Pax5*^{+/-} pro-B cells. The pro-B cells of a CD19⁻ *Pax5*^{+/-} clone were infected with a retrovirus expressing a truncated human (h) CD2 protein and then injected into 550 rad-irradiated *RAG2*^{-/-} mice. Three weeks after transfer, the thymus was analyzed by flow cytometry with PE-anti-hCD2, FITC-anti-CD4 and APC-anti-CD8 antibodies. The expression of CD4 and CD8 is shown for the hCD2⁺ thymocytes of donor origin. (F) Long-term engraftment of injected CD19⁻ *Pax5*^{+/-} pro-B cells in the bone marrow of *RAG2*^{-/-} mice. Eleven weeks after cell transfer, the bone marrow was analyzed by flow cytometry with PE-anti-hCD2, FITC-anti-CD19 and APC-anti-B220 antibodies. The expression CD19 and B220 is displayed for the hCD2⁺ cell of donor origin.

### Example 5

### Culture of human pro-B cells

Low-density mononuclear cells are prepared from human umbilical cord blood and enriched for CD34⁺ progenitor cells by MACS sorting with CD34 immunomagnetic beads (Miltenyi, Auburn, CA). The bead-sorted CD34⁺ cells (40-80% pure) are then stained with CyChrome-labeled anti-CD34, fluorescein isothiocyanate-coupled anti-CD10 and phycoerythrin-labeled anti-CD19 antibodies, and the CD34⁺CD19⁻CD10⁻ cells are sorted using a FACSVantage TSO flow-cytometer (Becton-Dickinson). The purified CD34⁺CD19⁻CD10⁻ cells are cultured in a pro-B cell medium on Petri dishes that are precoated with a confluent γ-irradiated monolayer of murine MS-5 stromal cells (Berardi et al, 1997). The pro-B cell medium consists of Iscove's Dulbecco modified medium (IMDM) containing 10% human AB serum (Institut Jacques BOY, Reims, France), 5% pre-tested fetal calf serum (HCC-6400; Stem Cell Technologies Inc., Vancouver, Canada) and the following recombinant (r) human (hu) cytokines: rhu-stem cell factor (SCF, 50 ng/ml; Amgen, CA, USA), rhu-interleukin (IL)-2 (5 ng/ml, Diaclone, France) and rhu-IL-15 (1 ng/ml; Diacone, France). The pro-B cells are cultured at 37°C in 5% CO₂ and fed every week by exchanging half of the medium.

### References

Adams B., et al., Genes Dev. 6, 1589-1607 (1992).
Agrawal S, and Zhao Q., Curr Opin Chem Biol; 2: 519-528, (1998)
Bai J, Rossi J, Akkina R., AIDS Res Hum Retroviruses, Mar 20;17(5):385-99, (2001).
Bain, G., et al., Cell 79, 885-892 (1994).
Bennett CF, Chiang MY, Chan H, Shoemaker JE, Mirabelli CK., Mol Pharmacol, Jun;41(6):1023-33 (1992).
Berardi, A. C., Meffre, E., Pflumio, F., Katz, A., Vainchenker, W., Schiff, C., and Coulombel, L., Blood 89, 3554-3564, (1997).
Birikh KR, Heaton PA, Eckstein F., Eur J Biochem; 245:1-16, (1997)
Björk P. and Kincade P. W., J. Immunol. 161, 5795-5799 (1998).
Braasch, DA and Corey DR, Biochemistry 41, 4503-4510, 2002
Brekken et al., Cancer Research 60:5117-5124, (2000).
Brummelkamp, T.R., Bernards, R., Agami, R., Science, Vol. 296, April 19, 5567:550-553 (2002)
Buchschacher, G. L., Jr., and Wong-Staal, F., Blood 95, 2499-2504, (2000).
Busslinger, M., Nutt, S. L., Rolink, A. G., Curr. Opin. Immunol. 12, 151-158, (2000).
Capaccioli S, Di Pasquale G, Mini E. et al., Biochem Biophys Res Commun; 197:818-825, (1993)
Childs, R. W., et al., J. Clinical Oncology 17, 2044-2049 (1999)
Childs, R., Chernoff, A., Contentin, N., Bahceci, E., Schrump, D., Leitman, S., Read, E. J., Tisdale, J., Dunbar, C., Linehan, W. M., Young, N. S., and Barrett, A. J., N. Engl. J. Med. 343, 750-758 (2000).
Cumano, A., Dorshkind, K., Gillis, S., and Paige, C. J., Eur. J. Immunol. 20, 2183-2189 (1990).
Deftos M. L., He Y.-W., Ojala E. W., Bevan M. J., Immunity 9, 777-786 (1998).
Drevs et al., Cancer Research 60:4819-4824, (2000).
Earnshaw DJ, Gait MJ., Antisense Nucleic Acid Drug Dev; 7:403-411, (1997)
Elbashir S.M., et al. Nature 411:494-498, (2001).
Emi, N., Friedmann, T., and Yee, J. K., J. Virol. 65, 1202-1207 (1991).
Fischer A., et al., Annu. Rev. Immunol. 15, 93-124 (1997).
Follenzi, A., and Naldini, L., Methods Enzymol. *346*, 454-465 (2002).
Galderisi U, Cascino A, Giordano A., J Cell Physiol; 181:251-257, (1999)
Gewirtz AM., Curr Opin Hematol; 5:59-71, (1998)
Hampel A., Prog Nucleic Acid Res Mol Biol; 58:1-39, (1998)
Hennequin et al., J. Med. Chem. 42:5369-5389, (1999)
Horcher, M., Souabni, A., Busslinger, M., Immunity 14, 779-790 (2001).
Irie A, Anderegg B, Kashani-Sabet M et al., Antisense Nucleic Acid Drug Dev; 9:341-349, (1999)
Kafri, T., van Praag, H., Ouyang, L., Gage, F. H., and Verma, I. M., J. Virol. 73, 576-584 (1999).
Kee, B. L., and Murre, C., J. Exp. Med. 188, 699-713 (1998).
Kondo, M., Weissman, I. L., Akashi, K., Cell 91, 661-672 (1997).
Kore A.R. and Eckstein F., Biochemistry; 38:10915-10918, (1999)
Kozmik Z., Wang S., Dörfler P., Adams B., Busslinger M., Mol. Cell. Biol. 12, 2662-2672 (1992).
Kurosaka, D., LeBien, T. W., and Pribyl, J. A. R., Exp. Hematol. *27*, 1271-1281, (1999)
LeBien T. W., Blood 96, 9-23 (2000).
Lever, A. M., Gene Ther 3, 470-471(1996)
Levine BL, Mosca JD, Riley JL, et al., Science, 272:1939 (1996).
Levine AM, Scadden DT, Zaia JA, Krishnan A., Hematology, Jan:463-78, (2001).
Lin, H. and Grosschedl, R., Nature 376, 263-267 (1995).
Lin et al., Cell Growth Differ. 9:49-58, (1998).
Luther-Wyrsch, A., Human Gene Therapy, 12, 377-389, (2001)
Mitsuyasu RT, Anton PA, Deeks SG, et al., Blood 96:785 (2000).
Montecino-Rodriguez E., Leathers H., Dorshkind K., Nature Immunol. 2, 83-88 (2001).
Morrison, S. J., et al., Cell 101, 499-510 (2000).
Naldini, L Blomer, U Gallay, P Ory, D Mulligan, R Gage, F H Verma, I M & Trono, D., Science 272, 263-267 (1996)
Namikawa, R., Muench, M. O., de Vries, J. E., and Roncarolo, M. G., Blood 87, 1881-1890, (1996)
Nishihara, M., Wada, Y., Ogami, K., Ebihara, Y., Ishii, T., Tsuji, K., Ueno, H., Asano, S., Nakahata, T., and Maekawa, T., Eur. J. Immunol. 28, 855-864, (1998)
Nutt, S. L., Urbánek, P., Rolink, A., Busslinger, M., Genes Dev. 11, 476-491 (1997).
Nutt S. L., Morrison A. M., Dörfler P., Rolink A., Busslinger M., EMBO J. 17, 2319-2333 (1998).
Nutt, S. L., Heavey, B., Rolink, A. G., Busslinger, M., Nature 401, 556-562 (1999a).
Nutt S. L., Rolink A. G., Busslinger M., Cold Spring Harb. Symp. Quant. Biol. 64, 51-59 (1999b).
Nutt S. L., et al., Nature Genet. 21, 390-395 (1999c).
Ogawa, M., Nishikawa, S., Ikuta, K., Yamamura, F., Naito, M., Takahashi, K., and Nishikawa, S.-I., EMBO J. 7, 1337-1343 (1988).
Poznansky MC, Evans RH, Foxall RB, et al., Nat Biotechnol. 18:729 (2000).
Presta et al., Cancer Research 57:4593-4599, (1997).
Quentmeier, H., Drexler, H. G., Fleckenstein, D., Zaborski, M., Armstrong, A., Sims, J. E., and Lyman, S. D., Leukemia *15*, 1286-1292 (2001).
Raport, C.J., Gosling, J., Schweickart, V.L., Gray, P.W. & Charo, I.F., J. Biol. Chem. 271, 17161-17166 (1996).
Rawlings, D. J., Quan, S. G., Kato, R. M., and Witte, O. N., Proc. Natl. Acad. Sci. USA 92, 1570-1574, (1995)
Rinkenberger J. L., Wallin J. J., Johnson K. W., Koshland M. E., Immunity 5, 377-386 (1996).
Rolink, A. G., Nutt, S. L., Melchers, F., Busslinger, M., Nature 401, 603-606 (1999).
Rosenberg ES, Billingsley JM, Caliendo AM, et al., Science, 278:1447, (1997).
Saleh et al., Cancer Research 56:393-401, (1996)
Schwenk, F., Kühn, R., Angrand, P.-O., Rajewsky, K., Stewart, A. F., Nucleic Acids Res. 26, 1427-1432 (1998).
Shinkai Y., et al., Cell 68, 855-867 (1992).
Sigvardsson, M., O'Riordan, M., Grosschedl, R., Immunity 7, 25-36 (1997).
Smith SM, Maldarelli F, Jeang KT., J Virol; 71:9713-9721, (1997)
Sui G, Soohoo C, Affar El B, Gay F, Shi Y, Forrester WC, Shi Y., Proc Natl Acad Sci U S A, Apr 16; 99(8):5515-20 (2002).
Urbánek, P., Wang, Z.-Q., Fetka, I., Wagner, E. F., Busslinger, M., Cell 79, 901-912 (1994).
Vaish NK, Kore AR, Eckstein F., Nucleic Acids Res; 26:5237-5242, (1998)
VandenDriessche, T., Naldini, L., Collen, D., and Chuah, M. K., Methods Enzymol. 346, 573-589 (2002)
Weissman, I. L., Cell 100, 157-168 (2000).
Wolf, M. L., Buckley, J. A., Goldfarb, A., Law, C. L., and LeBien, T., J. Immunol. *147*, 3324-3330, (1991)
Yang OO, Tran AC, Kalams SA, Johnson RP, Roberts MR, Walker BD., Proc Natl Acad Sci U S A., 94:11478, (1997).
Zhuang, Y., Soriano, P., Weintraub, H., Cell 79, 875-884 (1994).

## Claims

1. Pro-B cells of human origin that are deficient in functional Pax5 expression.

2. A method for producing human Pax5-deficient pro-B cells of claim 1, comprising the steps of
a) isolating mononuclear cells from human tissue,
b) enriching the cell population obtained in a) for lympoid progenitor cells,
c) in vitro differentiating these progenitors into pro-B cells and expanding the pro-B cells in culture with appropriate cytokines,
d) identifying and characterizing the pro-B cells according to the expression of B-lymphoid-specific cell surface proteins,
e) inactivating the *Pax5* gene in the cultured pro-B cells,
f) isolating and growing the Pax5-deficient pro-B cells under pro-B cell culture conditions.

3. The method of claim 2, wherein in step a) the tissue is selected from fetal liver, fetal cord blood, bone marrow and adult peripheral blood.

4. The method of claim 2, wherein in step b) lymphoid progenitor cells are enriched by sorting for cells co-expressing the antigens CD34, CD38 and CD10.

5. The method of claim 2, wherein in step c) the sorted CD34⁺CD38⁺CD10⁺ lymphoid progenitors are expanded and differentiated in vitro in co-culture with appropriate stromal cells in a pro-B cell medium.

6. The method of claims 2, wherein the pro-B cell medium contains recombinant human cytokines.

7. The method of claim 6, wherein the cytokines are stem cell factor (SCF), interleukin-2 (IL-2), interleukin-15 (IL-15) and thymic stromal lymphopoietin (TSLP).

8. The method of claim 2, wherein in step d) pro-B cells are identified by their expression of CD34⁺CD19⁺CD10⁺CD79a⁺CD38⁻.

9. The method of claim 8, wherein the pro-B cells are sorted by flow cytometric analysis using labeled anti-CD79a, anti-CD19,anti-CD34, anti-CD10 and anti-CD38 antibodies.

10. The method of claim 2, wherein in step e) expression of the *Pax5* gene is inactivated by means of antisense RNA.

11. The method of claim 2, wherein in step e) expression of the *Pax5* gene is inactivated by means of small interfering RNA.

12. The method of claim 2, wherein in step e) expression of the *Pax5* gene is inactivated by means of a ribozyme.

13. The method of any one of claims 10 to 12, wherein
i. an expression cassette encoding one or more Pax5-inhibiting oligonucleotide molecules is inserted into lentiviral vector,
ii. the lentiviral vector is transfected into specialized packaging cells to generate pseudotyped lentiviruses,
iii. pro-B cells obtained in step (c) are infected with the pseudotyped lentivirus obtained in ii. and cultured.

14. The method of claim 2, wherein in step f) the Pax5-deficient pro-B cells are identified by loss of expression of one or more Pax5 target genes.

15. The method of claim 14, wherein the Pax5-deficient pro-B cells are identified by loss of CD19 expression.

16. The method of claim 2, wherein pro-B cells lacking Pax5 expression are isolated by cell sorting and expanded in pro-B cell medium as in step c).

17. Autologous human Pax5-deficient pro-B cells or Pax5-deficient pro-B cells derived from a HLA-matched donor for use in the therapy of disorders associated with a depletion of the lymphoid system.

18. Autologous human Pax5-deficient pro-B cells or Pax5-deficient pro-B cells derived from a HLA-matched donor for the treatment of immunodeficiencies.

19. Autologous human Pax5-deficient pro-B cells or Pax5-deficient pro-B cells derived from a HLA-matched donor for the treatment of AIDS.

20. Pax5-deficient pro-B cells derived from a HLA-matched donor for use in the therapy of tumors.

21. Pro-B cells derived from a HLA-matched donor for use in non-myeloablative tumor therapy.

22. Pharmaceutical composition containing Pax5-deficient pro-B cells of human origin.

23. Pax5-deficient pro-B cells in which, in addition to Pax5, other functions are either partially suppressed or completely inactivated.
